# EUROPÄISCHE PATENTSCHRIFT

(11) **EP 2 073 890 B1**
(45) Veröffentlichungstag und Bekanntmachung des Hinweises auf die Patenterteilung: **17.02.2016**
(21) Anmeldenummer: 07818677.2
(22) Anmeldetag: 04.10.2007
(51) Int. Cl.: A61M 39/02, A61M 39/12

(54) **PORT FÜR EINEN KATHETER**
CATHETER PORT
POINT D'ENTRÉE DE CATHÉTER

(30) Priorität: 07.10.2006 DE 102006047519
(43) Veröffentlichungstag der Anmeldung: 01.07.2009
(60) Teilanmeldung: 12188556.0
(73) Patentinhaber: Fresenius Kabi Deutschland GmbH, 61352 Bad Homburg v.d.H. (DE)
(72) Erfinder: OSTER, Marcel, 41065 Mönchengladbach (DE); SCHUMACHER, Rainer, 65232 Taunusstein (DE)
(74) Vertreter: Maikowski & Ninnemann
(86) Internationale Anmeldenummer: PCT/EP2007/008598
(87) Internationale Veröffentlichungsnummer: WO 2008/040538

(56) Entgegenhaltungen:
- EP-A- 0 619 101
- WO-A-01/60444
- DE-C1- 4 129 782
- US-A- 5 213 574
- US-A- 5 460 612
- US-A- 5 718 682

## Beschreibung

Die Erfindung betrifft einen Port für einen Katheter zur Verabreichung eines Wirkstoffes zu einem entfernt liegenden Wirkort.

Zur Verabreichung von Wirkstoffen ist es bekannt, einen Port unter der Haut des Patienten zu implantieren, an den ein Katheter angeschlossen wird, der zu dem Wirkort geführt wird, an den der Wirkstoff gelangen soll. Der Port weist eine Kammer zur Aufnahme des Wirkstoffs auf, die von einer durchstechbaren Membran verschlossen ist, die unter der Haut liegt. Zum Befüllen der Kammer wird die Haut und die darunter liegende Membran mit einer Kanüle durchstochen und der Wirkstoff in die Kammer gespritzt. Von der Kammer gelangt der Wirkstoff dann über den Katheter zum Wirkort.

Die DE 41 29 782 C1 beschreibt einen Port, der ein Gehäuse mit einer unteren Ausnehmung zur Aufnahme des Wirkstoffs und einer oberen Ausnehmung zur Aufnahme der Membran aufweist. Die Membran wird in der Ausnehmung von einem Spannring gehalten, der einen Druck auf die Membran ausübt, so dass sich die Membran nach außen wölbt.

Aus der US-A-5 718 682 ist ein Port bekannt, dessen Gehäuse aus einem Ober- und Unterteil besteht, die unter Zwischenlage einer Membran miteinander verschraubt sind. Die Kammer zur Aufnahme des Wirkstoffs ist in dem Gehäuseunterteil ausgebildet, während das Gehäuseoberteil als Spannring dient.

Einen Port mit einem zweiteiligen Gehäuse, dessen Gehäuseunterteil als Kammer ausgebildet ist, beschreibt auch die US-A-4 772 270. Zwischen dem Gehäuseober- und -unterteil ist die Membran verklemmt.

Bei den bekannten Portsystemen stellt sich das Problem, dass das Gehäuseteil zur Aufnahme des Wirkstoffes aus einem für den Wirkstoff resistenten Material gefertigt sein muss, während das Gehäuseteil, das mit dem Patienten in Kontakt kommt, aus einem biokompatiblen Werkstoff bestehen muss. Insgesamt sollte der Port ein geringes Gewicht haben, und unempfindlich gegen Stöße und Schläge sein. Insbesondere die Kammer zur Aufnahme des Wirkstoffes sollte nicht durch eine abrutschende Kanüle beschädigt werden können.

Die US-A-6 213 973 schlägt vor, sämtliche Gehäuseteile aus einem biokompatiblen Kunststoff oder aus einem anderen biokompatiblen Material, beispielsweise Metall oder Keramik, herzustellen, das nicht von der Nadel zum Befüllen des Ports verletzt werden kann. Die Druckschrift schlägt alternativ aber auch vor, die Gehäuseteile aus unterschiedlichen Materialien zu fertigen.

Die WO 05/032645 beschreibt ein Portsystem mit einem implantierbaren Port und einem Katheter, bei dem die Kammer zur Aufnahme des Wirkstoffes in einem Einsatzstück ausgebildet ist, das in einer Ausnehmung eines einteiligen Gehäusekörpers unter klemmender Zwischenlage der Membran derart arretiert ist, dass das Einsatzstück einen Anpressdruck auf die Membran ausübt. Das Einsatzstück und das Gehäuse sind als Bajonettverschluss ausgebildet, so dass das Einsatzstück leicht in das Gehäuse eingesetzt und nach Schließen des Bajonettverschlusses sicher in dem Gehäuse fixiert ist. Das Einsatzstück sitzt derart in der Gehäuseausnehmung, dass dessen Unterseite bündig mit der Gehäuseunterseite abschließt.

Der Vorteil des aus der WO/05032645 bekannten Portsystem, das über eine Wirkstoffkammer verfügt, liegt darin, dass das Einsatzstück aus einem anderen Material als das Portgehäuse, beispielsweise Kunststoff oder Metall, insbesondere Titan oder Keramik, gefertigt werden kann, ohne dass der gesamte Port verändert zu werden braucht. Nachteilig ist jedoch der relativ aufwändige Bajonettverschluss.

Aus der US 5,213,574 ist ein Port für einen Katheter bekannt, bei dem ein Einsatzteil aus einem metallischen Material, vorzugsweise Titan, in einem Gehäuse 12 aus einem nichtmetallischen Material eingefasst ist.

Der Efindung liegt die Aufgabe zugrunde, einen einfach zu montierenden Port für einen Katheter zu schaffen, der die Fertigung der mit dem Wirkstoff in Kontakt kommenden Teile aus einem für den Wirkstoff resistenten Material erlaubt, während die mit dem Patienten in Kontakt kommenden Teile aus einem biokompatiblen Material bestehen können.

Die Lösung dieser Aufgabe erfolgt erfindungsgemäß mit den Merkmalen des Patentanspruchs 1. Bevorzugte Ausführungsformen der Erfindung sind Gegenstand der Unteransprüche.

Der erfindungsgemäße Port verfügt über ein zweiteiliges Gehäuse, in das ein Einsatzteil eingesetzt ist, in dem eine Kammer zur Aufnahme eines Wirkstoffes ausgebildet ist. Das Einsatzteil ist von dem Gehäuseoberteil unter Zwischenlage einer selbstabdichtenden Membran (Septum) klemmend in einer Gehäuseausnehmung des Gehäuseunterteils gehalten. Dabei ist das Einsatzteil durch die Gehäuseteile und die Membran vollständig nach außen abgeschirmt. Das Einsatzteil besitzt keine Kontaktfläche an der Außenhülle des Ports.

Das mit dem Patienten in Kontakt kommende Gehäuseober- und -unterteil ist aus einem leichteren biokompatiblen Material gefertigt, während das Einsatzteil unabhängig von dem Gehäuseober- und -unterteil aus einem für den Wirkstoff resistenten, möglicherweise schwereren Material besteht. Obwohl die mit dem Patienten in Kontakt kommenden Teile und die mit dem Wirkstoff in Berührung kommenden Teile des Port aus unterschiedlichen Materialien gefertigt sind, ist die Montage der aus unterschiedlichen Materialien bestehenden Teile des Ports einfach, da für den Zusammenbau lediglich das Einsatzteil in die Gehäuseausnehmung des Gehäuseunterteils eingesetzt und Gehäuseober- und -unterteil unter Zwischenlage der Membran miteinander verbunden werden brauchen, wodurch das Einsatzteil klemmend in der Gehäuseausnehmung gehalten wird.

Gehäuseober- und -unterteil bestehen aus einem biokompatiblen Kunststoff. Derartige Kunststoffe sind dem Fachmann bekannt, ein bevorzugter Kunststoff ist beispielsweise PEEK. Sie zeichnen sich nicht nur durch die Biokompatibilität, sondern auch gegenüber Metall oder Keramik durch ein geringeres Gewicht aus. Darüber hinaus können Gehäuseober- und -unterteil im Spritzgießverfahren einfach und mit freier Formgestaltung hergestellt werden, was bei Metall oder Keramikgehäusen nicht der Fall wäre.

Das Einsatzteil mit der Kammer zur Aufnahme des Wirkstoffs besteht hingegen aus einem keramischen Material. Derartige Materialien sind dem Fachmann bekannt. Sie zeichnen sich dadurch aus, dass sie gegenüber den Wirkstoffen grundsätzlich resistent sind und nicht von der Kanüle durchstochen werden können.

Der entscheidende Vorteil des erfindungsgemäßen Ports liegt darin, dass das Einsatzteil mit der Kammer zur Aufnahme des Wirkstoffes, das aus einem anderen Material als das Gehäuse besteht, vollständig von Gehäuse und Membran umschlossen wird, so dass das Einsatzteil nicht mit dem Patienten in Berührung kommt.

Eine weitere bevorzugte Ausführungsform der Erfindung sieht vor, dass Gehäuseober- und - unterteil miteinander verschweißt sind. Vorzugsweise werden die beiden Gehäuseteile im Ultraschall-Schweißverfahren fest miteinander verbunden. Dadurch wird eine sichere und reproduzierbare Verbindung erzielt, wobei sich die Prozessdaten des Schweißverfahrens für jeden Port leicht dokumentieren lassen. Es wird ein geschlossenes abgedichtetes System ohne Klebestellen geschaffen.

Bei einer weiteren bevorzugten Ausführungsform ist das Gehäuseoberteil in die Gehäuseausnehmung des Gehäuseunterteils eingesetzt. Damit wird das Gehäuseoberteil von dem Gehäuseunterteil umschlossen. Dies ist insofern vorteilhaft, als die konstruktionsbedingt von Gehäuseober- und -unterteil aufgenommene Membran einfach eingesetzt werden kann, ohne dass die Gefahr besteht, dass die Membran von den zu verbindenden Gehäuseteilen gequetscht wird. Es ist aber grundsätzlich auch möglich, dass das Gehäuseoberteil auf das Gehäuseunterteil aufgesetzt ist und das Gehäuseunterteil umschließt.

Das Gehäuseoberteil weist einen unteren und einen oberen Abschnitt auf, wobei der untere Abschnitt die Membran ringförmig umschließt und sich in eine Nut des Gehäuseunterteils erstreckt, während der obere Abschnitt einerseits die Membran übergreift und andererseits sich auf dem Gehäuseunterteil abstützt.

Da der sich in eine Nut des Gehäuseunterteils erstreckende untere Abschnitt des Gehäuseoberteils sich nicht auf dem Gehäuseunterteil abstützt, wird auch bei relativ großen Fertigungstoleranzen sichergestellt, dass der obere Abschnitt des Gehäuseoberteils unter Verpressung der Membran fest auf dem Gehäuseunterteil aufliegt.

Eine weitere bevorzugte Ausführungsform sieht vor, dass das Einsatzteil einen Flansch aufweist, mit dem das Einsatzteil auf einem Absatz in der Gehäuseausnehmung des Gehäuseunterteils aufliegt. Dadurch wird erreicht, dass sich das Einsatzteil in einem Bereich an dem Gehäuseunterteil abstützen kann, in dem das Gehäuseunterteil besonders dickwandig und stabil ausgebildet sein kann.

Das Einsatzteil ist vorzugsweise ein im Wesentlichen hohlzylindrischer Körper, das an der Unterseite mit einem Bodenteil verschlossen ist, während die Gehäuseausnehmung des Gehäuseunterteils vorzugsweise im Wesentlichen zylinderförmig ausgebildet ist. Es ist aber auch möglich, dass Einsatzteil und Gehäuseausnehmung nicht einen im Wesentlichen kreisförmigen, sondern elliptischen oder auch rechteckförmigen Querschnitt haben. Die im Wesentlichen rotationssymmetrische Ausbildung hat aber Vorteile bei der Fertigung und Handhabung.

Bei einer weiteren bevorzugten Ausführungsform ist das Einsatzteil verdrehsicher in das Gehäuseunterteil eingesetzt. Hierzu weist das Einsatzteil vorzugsweise einen nach außen vorspringenden Ansatz auf, der beim Einsetzen des Einsatzteils in die Gehäuseausnehmung in eine entsprechende Ausnehmung des Gehäuseunterteils eingreift. Beispielsweise kann der vorspringende Ansatz des Einsatzteils einen rechteckförmigen Querschnitt haben, der in eine nach oben offene Nut des Gehäuseunterteils eingeschoben ist. Dadurch ist das Einsatzteil leicht montierbar und verdrehsicher in das Gehäuseunterteil einsetzbar.

Zum Anschluss des Katheters an den erfindungsgemäßen Port ist vorzugsweise ein Anschlussstück vorgesehen, das vorzugsweise als eine sich durch eine Bohrung des Gehäuseunterteils erstreckende Kanüle ausgebildet ist.

Zur Abdichtung der Kanüle gegenüber dem Gehäuseunterteil sieht eine weitere bevorzugte Ausführungsform einen auf der Kanüle sitzenden Dichtring, vorzugsweise einen Silikon-Dichtring vor, der in einer Ausnehmung des Einsatzteils liegt. Die Kanüle ist vorzugsweise mit den übrigen Teilen des Ports verklebt und/oder verschweißt. An die Verklebung oder Verschweißung der Kanüle werden aber keine besonderen Anforderungen gestellt, da der Dichtring die Abdichtung übernimmt.

Im Folgenden wird ein Ausführungsbeispiel des erfindungsgemäßen Ports unter Bezugnahme auf die Zeichnungen näher erläutert.

Es zeigen:
- Fig. 1: Den erfindungsgemäßen Port in geschnittener Darstellung,
- Fig. 2: eine perspektivische Explosionsdarstellung des Gehäuseunterteils und des Einsatzteils des erfindungsgemäßen Ports in der Ansicht von der Seite und
- Fig. 3: eine perspektivische Explosionsdarstellung des Gehäuseunterteils und des Einsatzteils des erfindungsgemäßen Ports in der Ansicht von oben.

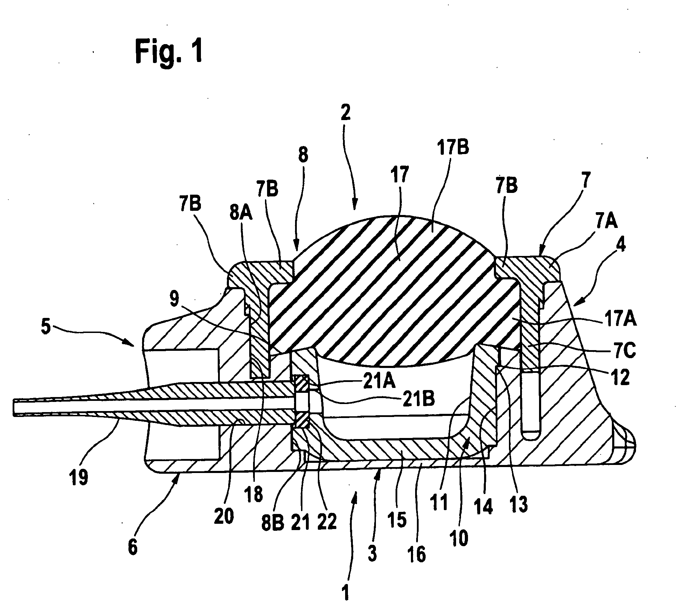

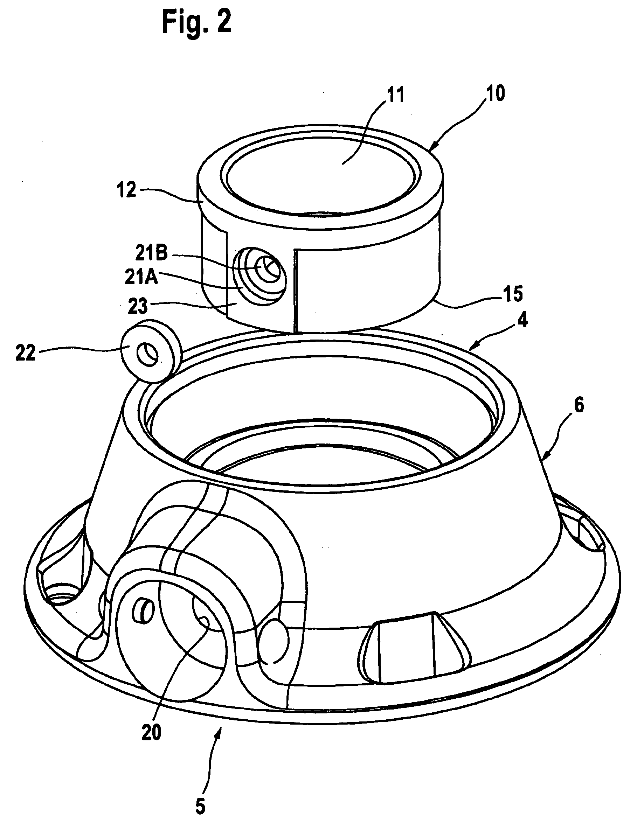

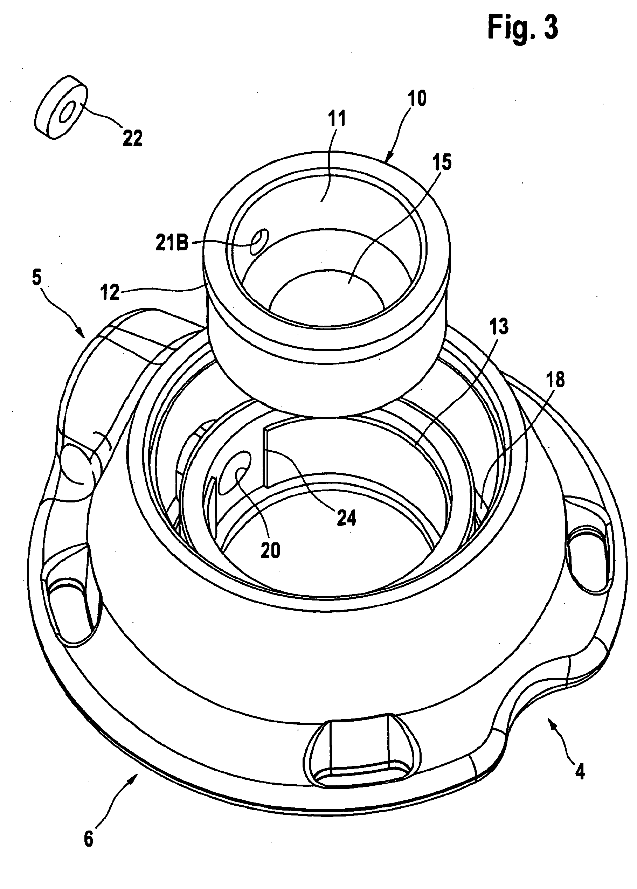

Figur 1 zeigt den erfindungsgemäßen Port in geschnittener Darstellung, während die Figuren 2 und 3 eine Explosionsdarstellung des Gehäuseunterteils und des Einsatzteils in vergrößerter Darstellung zeigen. Der Port, der etwa die Größe einer Fingerkuppe haben kann, weist ein flaches Gehäuse 1 auf, das unter der Haut des Patienten implantiert wird. Die unter der Haut liegende Oberseite des Portgehäuses 1 ist mit den Bezugszeichen 2, die Unterseite des Gehäuses mit 3, die Gehäusevorderseite mit 4 und die Gehäuserückseite mit 5 bezeichnet.

Das Gehäuse 1 ist zweiteilig ausgebildet. Es weist ein Gehäuseunterteil 6 und ein Gehäuseoberteil 7 auf. Gehäuseober- und -unterteil 6, 7 sind Spritzgießteile aus einem biokompatiblen Kunststoff.

Das Gehäuseunterteil 6 weist eine zentrale nach oben offene Ausnehmung 8 auf, die unter Ausbildung einer Stufe 9 einen oberen Bereich 8A mit einem größeren Durchmesser und einen unteren Bereich 8B mit einem kleineren Durchmesser hat. In den unteren Bereich 8B der Ausnehmung 8 ist passend ein im Wesentlichen zylindrisches Einsatzteil 10 eingesetzt, in dem eine nach oben offene Kammer 11 zur Aufnahme eines flüssigen Wirkstoffes ausgebildet ist. Dabei schließt die obere Seite des Einsatzteils 10 bündig mit der leicht schräg nach innen verlaufenden Stufe 9 ab.

Das Einsatzteil 10 ist ein im wesentlichen hohlzylindrischer Körper, der an der Unterseite mit einem Bodenteil 15 verschlossen ist. Es besteht aus einem metallischen oder keramischen Material, vorzugsweise einer Keramik, die für den Wirkstoff resistent ist. An seinem oberen Rand weist das Einsatzteil 10 einen Flansch 12 auf, mit dem sich das Einsatzteil an einem Absatz 13 abstützt, der an der Wandung 14 des unteren Bereichs 8B der Ausnehmung 8 ausgebildet ist. Zwischen dem Bodenteil 15 des Einsatzteils und dem Gehäuseboden 16 ist ein gewisses Spiel vorhanden, so dass das Einsatzteil 10 sich nicht an dem Gehäuseboden, sondern mit dem Flansch 12 in dem Bereich an dem Gehäuseunterteil 6 abstützt, in dem das Gehäuseunterteil dickwandig ausgebildet ist. Dadurch wird der Gehäuseboden entlastet.

In der Gehäuseausnehmung 8 sitzt oberhalb des Einsatzteils 10 eine mit einer Injektionskanüle durchstechbare, selbstabdichtende Membran 17 aus einem biokompatiblen Material. Das Gehäuseoberteil 7 hält die Membran 17 zusammen mit dem Einsatzteil 10 in der Gehäuseausnehmung 8 fest, welches in den oberen Bereich 8A der Gehäuseausnehmung 8 passend eingesetzt ist. Dabei übt die Membran 17 von oben einen Anpressdruck auf die Oberseite des Einsatzteils 10 aus, so dass das Einssatzteil fest in dem unteren Bereich 8B der Gehäuseausnehmung 8 sitzt.

Das Gehäuseoberteil 7 weist einen nach außen weisenden oberen Bereich 7A auf, mit dem sich das Gehäuseoberteil 7 an der Oberseite des Gehäuseunterteils 6 im Randbereich der Gehäuseausnehmung 8 abstützt. Mit einem nach innen weisenden oberen Abschnitt 7B übergreift das Gehäuseoberteil 7 den Randbereich der Membran 17, so dass die Membran gegen die Stufe 9 des Gehäuseunterteils 6 und die Oberseite des Einsatzteils 11 gepresst wird.

Darüber hinaus weist das Gehäuseoberteil 7 einen die Membran ringförmig umschließenden unteren Abschnitt 7C auf, der passend in dem oberen Bereich 8B der Gehäuseausnehmung 8 sitzt. Der untere Bereich 7C des Gehäuseoberteils 7 erstreckt sich in eine ringförmige Nut 18, die in dem Gehäuseunterteil 6 ausgebildet ist.

Die Membran 17 weist einen von dem Gehäuseoberteil 7 umschlossenen im Wesentlichen zylindrischen unteren Bereich 17A und einen durch Spannung erzeugten kalottenförmigen oberen Bereich 17B auf, der sich nach außen wölbt und frei liegt und von dem Gehäuseoberteil 7 ringförmig umschlossen wird.

Das Gehäuseoberteil 7 und das Gehäuseunterteil 6 sind nach dem Einsetzen des Einsatzteils 10 und der Membran 17 miteinander verschweißt, vorzugsweise mit einem Ultraschall-Schweißverfahren. Die konstruktionsbedingte Aufnahme der Membran 17 durch das Gehäuseober- und -unterteil 7, 6 verhindert, dass die Membran bei der Montage von den zu verschweißenden Teilen gequetscht wird. Vor der Montage werden die aus Peek bestehenden Einzelteile nach einem vorgegebenen Zyklus getempert, um den optimalen Kristallationszustand zu erhalten. Auf diese Weise besitzt die offene Kammer (11) keine gemeinsame Kontaktfläche mit den Gehäuseteilen (7, 6).

Zum Anschluss eines in den Figuren nicht dargestellten Katheters verfügt der Port über ein Anschlussstück 19, das als Kanüle ausgebildet ist. Die Kanüle 19 sitzt in einer querverlaufenden Bohrung 20 an der Rückseite 5 des Gehäuseunterteils 6 und erstreckt sich bis zu einer damit fluchtenden Bohrung 21 in dem Einsatzteil 10. Die Bohrung 21 des Einsatzteils 11 weist einen äußeren Abschnitt 21 A mit einem größeren Durchmesser und einen inneren Abschnitt 21B mit einem kleineren Durchmesser auf. Der Durchmesser des äußeren Abschnitts 21A ist derart bemessen, dass ein Dichtring, vorzugsweise ein Silikon-Dichtring 22 passend in die Bohrung eingesetzt werden kann, während der Durchmesser des inneren Abschnitts 21B kleiner als der Durchmesser des Dichtrings 22 und der Kanüle 19 ist. Der auf dem inneren Endstück der Kanüle 19 sitzende Dichtring 22, der in dem äußeren Abschnitt 21A der Bohrung 21 sitzt, dichtet die Kanüle 19 gegenüber dem Einsatzstück 10 ab. Auf diese Weise kommt bei bestimmungsgemäßem Gebrauch ein verabreichter Wirkstoff nur mit resistenten Materialien in Kontakt. Die Kanüle 19 ist weiterhin mit dem Gehäuseunterteil 20 verklebt, wobei die Verklebung eine zusätzliche Abdichtung schafft. An dem nach außen vorstehenden Abschnitt der Kanüle 19, welche verschiedene Geometrien aufweisen kann, so dass die bekannten in den Figuren nicht dargestellten Katheter auf die Kanüle aufgeschoben werden können. Eine mögliche Ausführungsform der Kanüle ist eine sich verjüngende, trompetenförmige Form.

An der der Rückseite 5 des Gehäuseunterteils 6 zugewandten Seite weist das Einsatzteil 10 einen nach außen vorspringenden Ansatz 23 mit rechteckförmigem Querschnitt auf, der sich vom Boden des Einsatzteils bis zu der Unterseite des Flansches 12 erstreckt. Der nach außen vorspringende Ansatz 23 sitzt in einer Ausnehmung 24, die in der Wandung des unteren Abschnitts 8B der Gehäuseöffnung 8 im Bereich der Bohrung 20 ausgebildet und zu der Stufe 9 hin offen ist. Dadurch ist es möglich, das Einsatzteil 10 mit dem vorspringenden Ansatz 23 von oben in die Nut 24 einzuschieben. Der passend in der Nut 24 sitzende Ansatz 23 des Einsatzteils 10 hält das Einsatzteil verdrehfest in der Gehäuseausnehmung 8 fest.

Zum Befüllen des Port wird die Membran 17 mit einer Injektionskanüle durchstochen, und ein flüssiger Wirkstoff wird in die Kammer 11 des Einsatzteils 10 gespritzt. Der Wirkstoff gelangt dann durch die Bohrung 21 in dem Einsatzteil 10 und die Kanüle 19 über den nicht dargestellten Katheter zu der Wirkstelle, die von dem Port entfernt liegt.

## Patentansprüche

1. Port für einen Katheter, mit
- einem Gehäuse (1), das eine Gehäuseausnehmung (8) aufweist,
- einem in der Gehäuseausnehmung (8) angeordneten Einsatzteil (10), in dem eine Kammer (11) zur Aufnahme eines Wirkstoffes ausgebildet ist,
- einer oberhalb der Kammer (11) in der Gehäuseausnehmung (8) angeordneten Membran (17) und
- einem mit der Kammer (11) in Flüssigkeitsverbindung stehenden Anschlussstück (19) für einen Katheter,
wobei das Gehäuse (1) zweiteilig aus einem Gehäuseoberteil (7) und einem Gehäuseunterteil (6) ausgebildet ist und das Einsatzteil (10) von dem Gehäuseoberteil (7) unter Zwischenlage der Membran (19) klemmend in der Gehäuseausnehmung (8) des Gehäuseunterteils (6) gehalten ist, so dass das Einsatzteil vollständig von dem Gehäuse (1) und der Membran (19) umschlossen ist,
**dadurch gekennzeichnet,**
**dass** das Gehäuseoberteil (7) und Gehäuseunterteil (6) aus einem anderen Material als das Einsatzteil (10) bestehen, indem das Gehäuseoberteil (7) und Gehäuseunterteil (6) aus einem biokompatiblen Kunststoff und das Einsatzteil (10) aus einem keramischen Material bestehen, wobei das Gehäuseoberteil (7) einen die Membran (17) ringförmig umschließenden unteren Abschnitt (7C) aufweist und der die Membran ringförmig umschließende untere Abschnitt (7C) des Gehäuseoberteils (7) sich in eine Nut (18) des Gehäuseunterteils (6) erstreckt.

2. Port nach Anspruch 1, **dadurch gekennzeichnet, dass** Gehäuseoberteil (7) und Gehäuseunterteil (6) miteinander verschweißt sind.

3. Port nach Anspruch 1 oder 2, **dadurch gekennzeichnet, dass** das Gehäuseoberteil (7) in die Gehäuseausnehmung (8) des Gehäuseunterteils (6) eingesetzt ist.

4. Port nach einem der Ansprüche 1 bis 3, **dadurch gekennzeichnet, dass** das Gehäuseoberteil (7C) einen die Membran (17) übergreifenden oberen Abschnitt (7B) aufweist.

5. Port nach einem der Ansprüche 1 bis 4, **dadurch gekennzeichnet, dass** das Gehäuseoberteil (7) einen sich auf dem Gehäuseunterteil (6) abstützenden oberen Abschnitt (7A) aufweist.

6. Port nach einem der Ansprüche 1 bis 5, **dadurch gekennzeichnet, dass** das Einsatzteil (10) einen Flansch (12) aufweist, mit dem das Einsatzteil auf einem Absatz (13) in der Gehäuseausnehmung (8) des Gehäuseunterteils (6) aufliegt.

7. Port nach einem der Ansprüche 1 bis 6, **dadurch gekennzeichnet, dass** das Einsatzteil (10) ein im wesentlichen hohlzylindrischer Körper ist, der mit einem Bodenteil (15) an der Unterseite verschlossen ist, und die Gehäuseausnehmung (8) des Gehäuseunterteils (6) im wesentlichen zylinderförmig ausgebildet ist.

8. Port nach einem der Ansprüche 1 bis 7, **dadurch gekennzeichnet, dass** die Membran (17) einen im wesentlichen zylindrischen unteren Abschnitt (17B), der von dem Gehäuseoberteil (7) ringförmig umschlossen ist, und einen im wesentlichen kalottenförmigen oberen Abschnitt (17A) aufweist, der frei liegt.

9. Port nach einem der Ansprüche 1 bis 8, **dadurch gekennzeichnet, dass** das Einsatzteil (10) verdrehsicher in das Gehäuseunterteil (6) eingesetzt ist.

10. Port nach einem der Ansprüche 1 bis 9, **dadurch gekennzeichnet, dass** das Einsatzteil (10) einen nach außen vorspringenden Ansatz (23) aufweist, der beim Einsetzen des Einsatzteils in die Gehäuseausnehmung (8) des Gehäuseunterteils (6) zur verdrehfesten Sicherung des Einsatzteils in der Gehäuseausnehmung in eine Ausnehmung (24) des Gehäuseunterteils eingreift.

11. Port nach einem der Ansprüche 1 bis 10, **dadurch gekennzeichnet, dass** das Gehäuseunterteil (6) eine Bohrung (20) aufweist, wobei das Anschlussstück für den Katheter als eine sich durch die Bohrung des Gehäuseunterteils erstreckende Kanüle (19) ausgebildet ist.

12. Port nach Anspruch 11, **dadurch gekennzeichnet, dass** die Kanüle (19) gegenüber dem Gehäuseunterteil (6) mit einem auf der Kanüle sitzenden Dichtring (22) abgedichtet ist, der in einer Ausnehmung (21A, 21B) des Einsatzteils (10) liegt.

13. Port nach einem der Ansprüche 1 bis 12, **dadurch gekennzeichnet, dass** das Einsatzteil (10) keine Kontaktfläche an der Außenhülle des Ports besitzt.

14. Port nach einem der Ansprüche 1 bis 12, **dadurch gekennzeichnet, dass** die offene Kammer zur Aufnahme des Wirkstoffs (11) keine gemeinsame Kontaktfläche mit dem Gehäuseoberteil (7) oder dem Gehäuseunterteil (6) besitzt.

## Claims

1. Catheter port with
- a housing (1), which has a housing recess (8),
- an insert part (10), which is arranged in the housing recess (8) and in which a chamber (11) for accommodating an active substance is formed,
- a membrane (17), which is arranged above the chamber (11) in the housing recess (8), and
- a connection piece (19) for a catheter, said connection piece (19) being fluidically connected to the chamber (11),
wherein the housing (1) is composed of two parts, namely a housing upper part (7) and a housing lower part (6), and the insert part (10) is held, clamped by the housing upper part (7) with the interposition of the membrane (19), in the housing recess (8) of the housing lower part (6), such that the insert part is completely enclosed by the housing (1) and the membrane (19),
**characterized in that**
the housing upper part (7) and housing lower part (6) are made from a different material than the insert part (10), with the housing upper part (7) and housing lower part (6) being made of a biocompatible plastic and the insert part (10) being made of a ceramic material, wherein the housing upper part (7) has a lower portion (7C) enclosing the membrane (17) in a ring shape, and the lower portion (7C) of the housing upper part (7) enclosing the membrane in a ring shape extends into a groove (18) of the housing lower part (6).

2. Port according to Claim 1, **characterized in that** housing upper part (7) and housing lower part (6) are welded to each other.

3. Port according to Claim 1 or 2, **characterized in that** the housing upper part (7) is inserted into the housing recess (8) of the housing lower part (6).

4. Port according to one of Claims 1 to 3, **characterized in that** the housing upper part (7C) has an upper portion (7B) engaging over the membrane (17).

5. Port according to one of Claims 1 to 4, **characterized in that** the housing upper part (7) has an upper portion (7A) resting on the housing lower part (6).

6. Port according to one of Claims 1 to 5, **characterized in that** the insert part (10) has a flange (12), with which the insert part lies on a shoulder (13) in the housing recess (8) of the housing lower part (6).

7. Port according to one of Claims 1 to 6, **characterized in that** the insert part (10) is a substantially hollow cylindrical body, which is closed on the underside by a floor part (15), and the housing recess (8) of the housing lower part (6) is substantially cylindrical.

8. Port according to one of Claims 1 to 7, **characterized in that** the membrane (17) has a substantially cylindrical lower portion (17B), which is enclosed in a ring shape by the housing upper part (7), and an upper portion (17A), which has substantially the shape of a spherical cap and lies free.

9. Port according to one of Claims 1 to 8, **characterized in that** the insert part (10) is inserted into the housing lower part (6) in a manner secure against rotation.

10. Port according to one of Claims 1 to 9, **characterized in that** the insert part (10) has an outwardly projecting extension (23) which, when the insert part is inserted into the housing recess (8) of the housing lower part (6), engages in a recess (24) of the housing lower part in order to secure the insert part in a manner secure against rotation in the housing recess.

11. Port according to one of Claims 1 to 10, **characterized in that** the housing lower part (6) has a bore (20), and the connection piece for the catheter is designed as a cannula (19) extending through the bore of the housing lower part.

12. Port according to Claim 11, **characterized in that** the cannula (19) is sealed off from the housing lower part (6) by a sealing ring (22) which sits on the cannula and which lies in a recess (21A, 21B) of the insert part (10).

13. Port according to one of Claims 1 to 12, **characterized in that** the insert part (10) has no contact surface on the outer shell of the port.

14. Port according to one of Claims 1 to 12, **characterized in that** the open chamber for accommodating the active substance (11) has no common contact surface with the housing upper part (7) or the housing lower part (6).

## Revendications

1. Raccord pour un cathéter avec
- un boîtier (1) qui comprend un évidement de boîtier (8),
- un insert (10) disposé dans un évidement de boîtier (8), dans lequel une chambre (11) est prévue pour le logement d'un principe actif,
- une membrane (17) disposée au-dessus de la chambre (11) dans l'évidement de boîtier (8) et
- un élément de raccordement (19) pour un cathéter, en liaison fluidique avec la chambre (11),
le boîtier (1) étant constitué de deux éléments : une partie supérieure de boîtier (7) et une partie inférieure de boîtier (6) et l'insert (10) étant maintenu par serrage par la partie supérieure du boîtier (7), par l'intermédiaire de la membrane (17) dans l'évidement (8) de la partie inférieure du boîtier (6), de façon à ce que l'insert soit entièrement entouré par le boîtier (1) et la membrane (17),
**caractérisé en ce que**
la partie supérieure du boîtier (7) et la partie inférieure du boîtier (6) sont constituées d'un matériau différent de l'insert (10), du fait que la partie supérieure du boîtier (7) et la partie inférieure du boîtier (8) sont constituées d'une matière plastique biocompatible et l'insert (10) est constitué d'un matériau céramique, la partie supérieure du boîtier (7) comprenant une portion inférieure (7C) entourant de manière annulaire la membrane (17) et la portion inférieure (7C) de la partie supérieure du boîtier (7), entourant de manière annulaire la membrane, s'étendant dans une rainure (18) de la partie inférieure du boîtier (6).

2. Raccord selon la revendication 1, **caractérisé en ce que** la partie supérieure du boîtier (7) et la partie inférieure du boîtier (6) sont soudées entre elles.

3. Raccord selon la revendication 1 ou 2, **caractérisé en ce que** la partie supérieure du boîtier (7) est insérée dans l'évidement de boîtier (8) de la partie inférieure du boîtier (6).

4. Raccord selon l'une des revendications 1 à 3, **caractérisé en ce que** la partie supérieure du boîtier (7C) comprend une portion supérieure (7B) qui vient en prise avec la membrane (17).

5. Raccord selon l'une des revendications 1 à 4, **caractérisé en ce que** la partie supérieure du boîtier (7) comprend une portion supérieure (7A) s'appuyant sur la partie inférieure du boîtier (6).

6. Raccord selon l'une des revendications 1 à 5, **caractérisé en ce que** l'insert (10) comprend une bride (12) avec laquelle l'insert repose sur un épaulement (13) dans l'évidement du boîtier (8) de la partie inférieure du boîtier (6).

7. Raccord selon l'une des revendications 1 à 6, **caractérisé en ce que** l'insert (10) est un corps creux essentiellement cylindrique qui est obturé avec une partie de fond (15) au niveau de la partie inférieure et l'évidement de boîtier (8) de la partie inférieure du boîtier (6) est essentiellement cylindrique.

8. Raccord selon l'une des revendications 1 à 7, **caractérisé en ce que** la membrane (17) comprend une portion inférieure (17B) essentiellement cylindrique, qui est entourée de manière annulaire par la partie supérieure du boîtier (7), et une portion supérieure (17A) essentiellement en forme de calotte, qui est libre.

9. Raccord selon l'une des revendications 1 à 8, **caractérisé en ce que** l'insert (10) est inséré avec un blocage en rotation dans la partie inférieure du boîtier (6).

10. Raccord selon l'une des revendications 1 à 9, **caractérisé en ce que** l'insert (10) comprend un épaulement (23) en saillie vers l'extérieur qui, lors de l'insertion de l'insert dans l'évidement de boîtier (8) de la partie inférieure du boîtier (6) pour le blocage anti-rotation de l'insert dans l'évidement du boîtier, s'emboîte dans un évidement (24) de la partie inférieure du boîtier.

11. Raccord selon l'une des revendications 1 à 10, **caractérisé en ce que** la partie inférieure du boîtier (6) présente un alésage (20), l'élément de raccordement pour le cathéter étant conçu comme une canule (19) s'étendant à travers l'alésage de la partie inférieure du boîtier.

12. Raccord selon la revendication 11, **caractérisé en ce que** la canule (19) est étanchéifiée par rapport à la partie inférieure du boîtier (6) avec une bague d'étanchéité (22) posée sur la canule, qui se trouve dans un évidement (21A, 21B) de l'insert (10).

13. Raccord selon l'une des revendications 1 à 12, **caractérisé en ce que** l'insert (10) comprend une surface de contact sur l'enveloppe externe du raccord.

14. Raccord selon l'une des revendications 1 à 12, **caractérisé en ce que** la chambre ouverte ne comprend, pour le logement du principe actif (11), aucune surface de contact commune avec la partie supérieure du boîtier (7) ou la partie inférieure du boîtier (6).
